# EUROPEAN PATENT SPECIFICATION

(11) **EP 1 468 992 B1**
(45) Date of publication and mention of the grant of the patent: **07.11.2007**
(21) Application number: 04008417.0
(22) Date of filing: 07.04.2004
(51) Int. Cl.: C07D 223/26

(54) **A process for the preparation of opipramol**
Verfahren zur Herstellung von Opipramol
Procédé pour la préparation d'opipramol

(30) Priority: 18.04.2003 IT mi20030828
(43) Date of publication of application: 20.10.2004
(73) Proprietor: FARCHEMIA S.r.l., 24047 Treviglio (BG) (IT)
(72) Inventor: Breviglieri, Gabriele, 24047 Treviglio (BG) (IT); Contrini, Sergio, 24047 Treviglio (BG) (IT); Assanelli, Cinzia, 24047 Treviglio (BG) (IT)
(74) Representative: Bianchetti, Giuseppe

(56) References cited:
- EP-A- 0 793 652
- GB-A- 921 961

## Description

The present invention relates to a process for the preparation of N-[3-[4-(2-hydroxyethyl)piperazino]propyl]-iminostilbene, of formula (I) 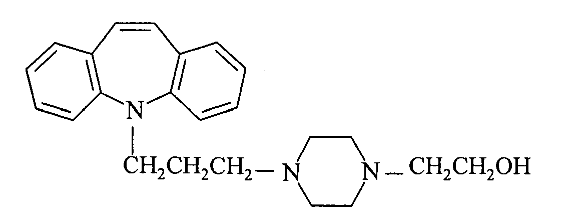 known as opipramol, which is an antidepressive and antipsychotic drug.

### Technological background

A number of processes for the preparation of opipramol (see FR M209, CH 359 143, CH 360 061, DE 1 133 729) are known, usually based on the N-alkylation of iminostilbene (II) with 1-bromo-3-chloropropane and the subsequent reaction of the resulting N-(3-alogenopropyl)iminostilbene (III) (halogen being chlorine or bromine) with N-(2-hydroxyethyl)piperazine.

According to the already cited DE 1 133 729, iminostilbene alkylation involves the use of a strong base (namely sodium amide) to obtain from iminostilbene the corresponding anion which is subsequently N-alkylated. This reaction however involves the simultaneous, partial formation of N-allyliminostilbene (IV), according to the following scheme:

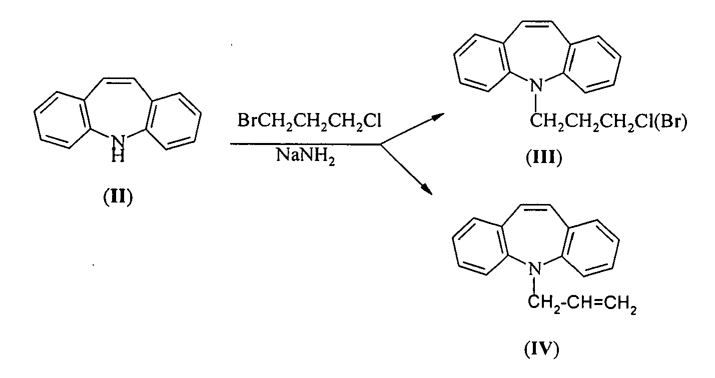

According to Journal of Heterocyclic Chemistry, 19, 1569-71 (1982), iminostilbene can be reacted with 1-bromo-3-chloropropane in the presence of aqueous sodium hydroxide and a phase transfer catalyst. It has also been observed that under these conditions undesired impurities form, due to the presence of sodium hydroxide, which is also a strong base.

More recently, EP 0 793 652 disclosed a process for the preparation of N-(3-halopropyl)iminostilbene (III) by *"reacting iminostilbene with 1-bromo-3-chloropropane in the presence of a weak base selected from a hydrogen phosphate salt of the formula MₓHPO₄ and an acetate salt of the formula (CH₃COO)ₓM, wherein M is an alkali metal, alkaline earth metal or ammonium and x is 1 or 2, depending on the valence of M, in the presence of a phase transfer agent and in a solvent substantially inert to the reaction reagents and removing solvent, any excess 1-bromo-3-chloropropane and solids present in the reaction mixture to obtain N-(3-halopropyl) iminostilbene* ".

EP 0 793 652 also claims a process for the preparation of opipramol in which, previous separation of any unreacted 1-bromo-3-chloropropane and salts, N-(3-halopropyl)iminostilbene obtained as above is directly reacted, without intermediate isolation, with N-(2-hydroxyethyl)piperazine in the presence of a weak base, to give opipramol.

EP 0 793 652 mentions disodium or dipotassium hydrogen phosphate as weak bases for the reaction between iminostilbene and 1-bromo-3-chloropropane; an ammonium quaternary salt, particularly benzyltrimethyl ammonium, as phase transfer agent; and toluene as the solvent. Such process is undoubtedly an improvement compared with those previously known. However, both yields and purity of the final product have still to be improved, and the use of high amounts of toluene and Isopar (high boiling hydrocarbon) is necessary and operatively complex.

### Summary of the invention

It has now been found that N-(3-halopropyl)iminostilbene (III) can be obtained in a very simple manner by reaction of iminostilbene with a 1-bromo-3-chloropropane excess in the presence of potassium carbonate, a small amount of polyethylene glycol 6000 and a small amount of water, in the absence of solvents (except for 1-bromo-3-chloropropane), washing of the reaction mixture with water and removal of the bromochloropropane excess, and by direct reaction of the resulting intermediate (III) with N-(2-hydroxyethylpiperazine) in the absence of other bases. It has been found that water, even in very small amounts, is indispensable to prevent formation of impurities hard to identify.

### Disclosure of the invention

According to the invention, iminostilbene is reacted with a 1-bromo-3-chloropropane excess (approx. 3.5-4.5 moles per mole of iminostilbene) and with approx. 1 mole of potassium carbonate per mole of iminostilbene, in the presence of approx. 10-15% by weight (referred to iminostilbene) of both PEG 6000 and water. The 1-bromo-3-chloropropane is excess of preferably 3.7-4 moles per mole of iminostilbene, while both PEG 6000 and water are both used amounts of approx. 12.5% by weight, referred to iminostilbene. The mixture is heated at 80-100°C, preferably 90-95°C, for 20-30 hours, then cooled to 40-60°C and added with approx. 40-60 parts by weight of water per 100 parts by weight of the starting mixture. After that, the lower organic phase is separated, keeping this temperature, and the higher aqueous phase is extracted with some chloroform, which is then added to the organic phase. Finally chloroform is evaporated off and the diahalopropane excess is recovered by distillation under slight vacuum (about 15 mm/Hg). The dihalopropane, consisting of 1-bromo-3-chloropropane, 1,3-dibromopropane and 1,3-dichloropropane, is recycled. The residue is directly reacted with 2-2.5 moles of N-(2-hydroxyethyl)piperazine per mole of starting iminostilbene, in an inert solvent, preferably toluene, without addition of other bases. The mixture is heated at 100-120°C for 18-30 hours, then worked up according to conventional procedures.

The process of the invention is further illustrated by the following example.

### Example

A mixture of
40 kg of iminostilbene,
120 kg of 1-bromo-3-chloropropane,
30 kg of potassium carbonate,
5 kg of PEG 6000 and
5 kg of water
is heated for 24 hours at 90-95°C. The mixture is then cooled to about 50°C, added with 100 liters of water, under stirring, the phases are decanted, the lower organic phase is recovered and the aqueous phase is extracted with 20 liters of chloroform, which is then added to the organic phase. Chloroform is evaporated off under atmospheric pressure, then dihalopropanes are distilled under about 15 mm/Hg (60 to 65 kg) for recycle. The residue is taken up in 150 liters of toluene, refluxed with 59 kg of N-(2-hydroxyethyl)piperazine for 24 hours, then cooled to 40-50°C and washed under stirring with 50 liters of water. The aqueous phase is discarded. The organic phase is added with a mixture of 100 liters of water and 20 liters of 35% hydrochloric acid to pH 3.5-4, under stirring. The phases are separated, the organic one is discarded and the aqueous phase is added with 100 liters of chloroform, then with ammonia to pH 9, thereby extracting opipramol base, from which the dihydrochloride is obtained with known methods. M.p. 228-230°C, (HPLC) purity above 99%. Yield 87%.

## Claims

1. A process for the preparation of N-[3-[4-(2-hydroxyethyl)piperazino]propyl]-iminostilbene of formula 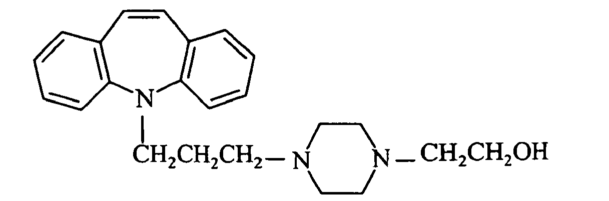 **characterized in that**
a) iminostilbene is reacted with a 1-bromo-3-chloropropane excess, in the presence of potassium carbonate, PEG (polyethylene glycol) 6000 and water, and
b) the resulting mixture of N-(3-chloropropyl)- and N-(3-bromopropyl)-iminostilbene, after removing the inorganic salts and recovering the 1,3-dihalopropanes excess, is directly reacted with N-(2-hydroxyethyl)piperazine in toluene, in the absence of other bases.

2. A process according to claim 1, **characterized in that** 3.5 to 4.5 moles of 1-bromo-3-chloropropane per mole of iminostilbene are used.

3. A process according to the above claims, **characterized in that** 3.7 to 4 moles of 1-bromo-3-chloropropane per mole of iminostilbene are used.

4. A process according to the above claims, **characterized in that** approx. 1 mole of potassium carbonate per mole of iminostilbene is used.

5. A process according to the above claims, **characterized in that** both PEG 6000 and water are present in amounts of 10-15% by weight on the iminostilbene weight.

6. A process according to the above claims, **characterized in that** both PEG 6000 and water are present in amounts of 12.5% by weight on the iminostilbene weight.

7. A process according to the above claims, **characterized in that** reaction a) is carried out by heating to 90-95°C for 20-30 hours.

8. A process according to the above claims, **characterized in that** the mixture of N-(3-halopropyl)iminostilbenes is reacted with 2-2.5 moles of N-(2-hydroxyethyl)piperazine per mole of starting iminostilbene.

## Patentansprüche

1. Ein Verfahren für die Herstellung von N-[3-[4-(2-Hydroxyethyl)piperazin]propyl]-iminostilben der Formel 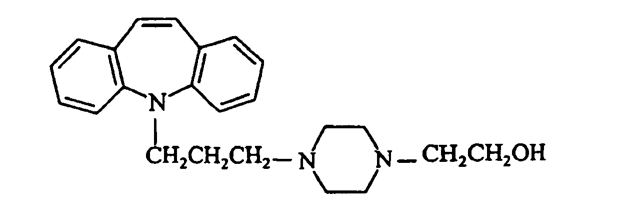 **dadurch gekennzeichnet, dass**
a) Iminostilben mit einem Überschuss von 1-Brom-3-chlorpropan in der Gegenwart von Kaliumcarbonat, PEG (Polyethylenglykol) 6000 und Wasser reagieren gelassen wird, und
b) die resultierende Mischung von N-(3-Chlorpropyl)- und N-(3-Brompropyl)-iminostilben nach dem Entfernen der anorganischen Salze und Rückgewinnen des Überschusses an 1,3-Dihalopropanen unmittelbar mit N-(2-Hydroxyethyl)piperazin in Toluol in der Abwesenheit von anderen Basen reagieren gelassen wird.

2. Ein Verfahren nach Anspruch 1, **dadurch gekennzeichnet, dass** 3,5 bis 4,5 Mol an 1-Brom-3-chlorpropan pro Mol an Iminostilben verwendet werden.

3. Ein Verfahren nach den vorherigen Ansprüchen, **dadurch gekennzeichnet, dass** 3,7 bis 4 Mol an 1-Brom-3-chlorpropan pro Mol an Iminostilben verwendet werden.

4. Ein Verfahren nach den vorherigen Ansprüchen, **dadurch gekennzeichnet, dass** ungefähr 1 Mol an Kaliumcarbonat pro Mol an Iminostilben verwendet wird.

5. Ein Verfahren nach den vorherigen Ansprüchen, **dadurch gekennzeichnet, dass** PEG 6000 und Wasser beide in Mengen von 10 bis 15 Gew.-%, bezogen auf das Iminostilben-Gewicht, zugegen sind.

6. Ein Verfahren nach den vorherigen Ansprüchen, **dadurch gekennzeichnet, dass** PEG 6000 und Wasser beide in Mengen von 12,5 Gew.-%, bezogen auf das Iminostilben-Gewicht, zugegen sind.

7. Ein Verfahren nach den vorherigen Ansprüchen, **dadurch gekennzeichnet, dass** die Reaktion a) durch Erwärmen auf 90 bis 95 °C für 20 bis 30 Stunden durchgeführt wird.

8. Ein Verfahren nach den vorherigen Ansprüchen, **dadurch gekennzeichnet, dass** die Mischung von N-(3-Halopropyl)iminostilbenen mit 2 bis 2,5 Mol an N-(2-Hydroxyethyl)piperazin pro Mol an Ausgangs-Iminostilben reagieren gelassen wird.

## Revendications

1. Procédé de préparation de N-[3-[4-(2-hydroxyéthyl)pipérazino]propyl]-iminostilbène de formule 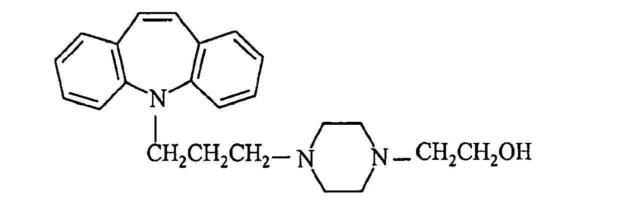 **caractérisé en ce que**
a) l'iminostilbène est mis à réagir avec un excès de 1-bromo-3-chloropropane, en présence de carbonate de potassium, de PEG (polyéthylène glycol) 6000 et d'eau, et
b) le mélange résultant de N-(3-chloropropyl)- et N-(3-bromopropyl)-iminostilbène, après élimination des sels inorganiques et récupération des 1,3-dihalogénopropanes en excès, est directement mis à réagir avec de la N-(2-hydroxyéthyl)pipérazine dans du toluène, en l'absence d'autres bases.

2. Procédé selon la revendication 1, **caractérisé en ce que** de 3,5 à 4,5 moles de 1-bromo-3-chloropropane par mole d'iminostilbène sont utilisées.

3. Procédé selon les revendications ci-dessus, **caractérisé en ce que** de 3,7 à 4 moles de 1-bromo-3-chloropropane par mole d'iminostilbène sont utilisées.

4. Procédé selon les revendications ci-dessus, **caractérisé en ce qu'**environ 1 mole de carbonate de potassium par mole d'iminostilbène est utilisée.

5. Procédé selon les revendications ci-dessus, **caractérisé en ce que** du PEG 6000 et de l'eau sont tous deux présents en des quantités de 10 à 15 % en poids par rapport au poids d'iminostilbène.

6. Procédé selon les revendications ci-dessus, **caractérisé en ce que** du PEG 6000 et de l'eau sont tous deux présents en des quantités de 12,5 % en poids par rapport au poids d'iminostilbène.

7. Procédé selon les revendications ci-dessus, **caractérisé en ce que** la réaction a) est réalisée par chauffage à 90 à 95 °C pendant 20 à 30 heures.

8. Procédé selon les revendications ci-dessus, **caractérisé en ce que** le mélange de N-(3-halogénopropyl)iminostilbènes est mis à réagir avec de 2 à 2,5 moles de N-(2-hydroxyéthyl)pipérazine par mole d'iminostilbène de départ.
